# EUROPEAN PATENT SPECIFICATION

(11) **EP 4 069 329 B1**
(45) Date of publication and mention of the grant of the patent: **10.01.2024**
(21) Application number: 20830303.2
(22) Date of filing: 30.11.2020
(51) Int. Cl.: A61M 1/36, B01L 3/00, A61M 1/38, A61J 1/10

(54) **CENTRIFUGE TUBE, CENTRIFUGE ADAPTER, DEVICE FOR THE PRODUCTION OF A PRP-CONTAINING MEDICAMENT AND SINGLE-USE KIT FOR MANUAL PRODUCTION OF PRP**
ZENTRIFUGENRÖHRCHEN, ZENTRIFUGENADAPTER, VORRICHTUNG ZUR HERSTELLUNG EINES PRP-ENTHALDENTEN ARZNEIMITTELS UND KIT FÜR DEN EINMALGEBRAUCH ZUR MANUELLEN FERTIGUNG VON PRP
TUBE DE CENTRIFUGATION, ADAPTATEUR POUR UNE CENTRIFUGEUSE, DISPOSITIF POUR LA PRODUCTION D'UN MÉDICAMENT CONTENANT PRP ET KIT POUR LA PRODUCTION MANUELLE DE PRP À L'USAGE UNIQUE

(30) Priority: 04.12.2019 IT 201900022947
(43) Date of publication of application: 12.10.2022
(73) Proprietor: Prometheus S.r.l., 43122 Parma (IT)
(72) Inventor: DELLA RAGIONE, Riccardo, 43122 Parma (IT); MICHELANGELI, Alice, 43122 Parma (IT); MENOZZI, Valentina, 63824 Altidona (FM) (IT)
(74) Representative: Dall'Olio, Christian
(86) International application number: PCT/IB2020/061274
(87) International publication number: WO 2021/111274

(56) References cited:
- EP-B1- 2 544 697
- US-A1- 2002 185 186
- US-A1- 2016 000 062
- US-B2- 9 696 242

## Description

### FIELD OF THE INVENTION

The present invention relates to the technical sector of methods and means of production of a medication utilisable for the treatment of skin lesions and osteochondral or joint pathologies, and in particular for the regeneration of tissues.

### DESCRIPTION OF THE PRIOR ART

The medication is obtained by combining a blood derivative with suitable biomaterials that are bioresorbable. When of an autologous type, the medication is created only at point of need, following a collection of blood from the patient to be treated. The blood derivative contained therein is a platelet-rich plasma (thrombocytes) which is also called PRP, using the acronym. The PRP is obtained by centrifugation of blood and has a high concentration of growth factors. For this reason it has high effectiveness in the regeneration of tissues. Obviously PRP has a concentration of platelets greater than that of the blood plasma from which it has been obtained; typically that concentration is 4x-6x, preferably 6x-9x. To obtain the PRP, a procedure can be operated that includes a single stage of centrifugation of the blood in which, the red blood cells (or erythrocytes) on the bottom of the receptacle of centrifugation deposit, and wherein the relative supernatant has a variable composition. This is because, in a first region, in proximity of the sediment of red blood cells, the supernatant has a greater concentration of platelets, while in a second region above the first region, it has a lower concentration of platelets. Therefore, the supernatant is first collected from the second region (which is poor in platelets) and discarded, and then the supernatant is collected from the first region, thus obtaining the PRP. It is necessary to collect as much PRP as possible, without also collecting the red blood cells. In fact, the presence thereof in the medication determines a negative effect on the relative effectiveness. In this matter, to facilitate the collection of the PRP, the container (which can be a test tube or a vial) for centrifuging, in which the blood is transferred, can include a narrowing in a relative vertical section. For the interface between the sediment of red blood cells and the first region to be at the position of the narrowing, it is necessary to carry out a collection of a precise quantity of blood which must have a composition that is not far removed from the standard composition. Alternatively, to obtain the PRP, a procedure can be included that includes two successive stages of centrifugation. In the first stage, a first sterile container is used to centrifuge the collected blood so that the red blood cells deposit on the bottom and a first supernatant, free of red blood cells, is collected. Thereafter, in a different sterile container, the first supernatant is centrifuged so that the platelets are deposited on the bottom, and a second supernatant is collected, known as platelet-poor plasma or "PPP" and the PPP is added for a volume of about 10% of the collected volume of whole blood to the platelets deposited on the bottom so as to re-suspend the platelets, obtaining the platelet-rich plasma, in which the platelets must be vital and distributed as homogeneously as possible, while conserving the sterility thereof. This can be done under a fume hood with a laminar flow of sterile air using a laboratory pipette and opening the container, or vigorously shaking the closed container. In the last modality, the PRP obtained is not homogeneous but has platelet aggregates. Consequently, the medication obtainable therewith will have an application and effectiveness that are not uniform. In relation to the resuspension under the fume hood, not all departments of a hospital, clinics and veterinary surgeries have a fume hood available.

In the medical field, a connector called a "needle free connector" is known, which is interposable between a medical device for collection and/or storage and a manual collecting and injecting medical device of a liquid to place the two devices in fluid communication without altering the relative sterility thereof. The first of these can be a medical container for collecting or storing a biological liquid (blood, plasma, urine etc..) or a medical liquid (saline solution, pharmacological system etc.). The collection and injection device can comprise a cylinder (62)-piston (61) system and relative connection means, and typically a needle-free syringe. The needle free connector, at a first longitudinal end thereof, is engageable with an opening of the medical device for collection and/or storage and defines internally thereof a channel having a relative longitudinal axis and comprises, at a second longitudinal end, a compression valve. The compression valve is constituted by an elastically longitudinally-deformable element respective to the axis thereof. The connector is conformed so that, when the second longitudinal end is free, the valve obstructs the channel, preventing the passage of liquids and airborne bodies. The needle free connector is also conformed so that, when the second longitudinal end of the needle free connector engages with the connecting means of the manual collecting and injecting medical device, the valve is compressed, opening the channel and enabling a liquid to flow. The above-described centrifugation steps are carried out with a bench centrifuge. It includes at least two centrifuge stations (to balance the load during the centrifugation) in which the containers for the bench centrifuge are inserted, which typically are constituted by test tubes or vials and optionally a relative cap.

Typically, to realise the medications comprising PRP, the PRP is mixed with a gelling agent and optionally the gel obtained thus can be supported on a support layer made of a bio-resorbable and porous material also known as a "scaffold"; this simplifies the topical application of the medication.

From the above, in relation to the means and methods of production of the medication, the need to manually produce a medication comprising PRP strongly emerges in which the platelets are uniformly diffused, without any need to operate under a fume hood to maintain the sterility of the PRP and the medication with simple procedures and without expensive equipment. Further, an especially known need is to minimise the possibility of contamination of the PRP produced and thus the medication obtained using the PRP, especially when a medication in gel form is obtained following the addition of calcium salts and incubation for at least 30 minutes in order to obtain the gelling of the PRP; since if this process took place in an environment that was not closed and sterile, the risk of contamination of the PRP with particles and micro-organisms would be high.

US9696242A1 discloses a centrifuge tube with a cap which has a venting port, a fluid delivery port and a fluid withdrawal port configured to engage with a tubing segment that extends from said port towards the bottom of the tubing segment.

### SUMMARY OF THE INVENTION

The aim of the present invention consists in reducing and/or obviating the above-cited disadvantages with respect to the means and method of production of a medication comprising platelet-rich plasma.

In particular, the present invention has the aim of being able to produce PRP in which the platelets are re-suspended homogeneously and, consequently, also a medication which comprises it avoiding external contaminations without having to operate under a fume hood, using laboratory centrifuges of a known type and with modest costs. A further aim of the invention is to provide means and methods for obtaining the PRP and the medication produced with the PRP by using a closed and sterile hydraulic circuit with the aim of excluding any form of contamination due to the opening of a hydraulic circuit or a container.

These aims and objectives are attained by a separation and a manual production method of platelet-rich plasma as claimed in the following independent claims. An adapter device for centrifuge stations of bench centrifuges, a production device of a medication comprising platelet-rich plasma, , and a manual production method of a medication are herein disclosed. The present invention enables being able to use a common bench centrifuge utilisable in the above-mentioned and known PRP production procedures that can be used in the production of a medication which comprises PRP. It is clear that, according to the invention, it is possible to produce the PRP and the medication in a closed circuit and in conditions that prevent contamination thereof. Therefore, the presence of a fume hood is superfluous.

Note that, thanks to the adapter device it is possible to centrifuge the collected blood directly in a medical bag for collecting biological liquids in which the collected blood is stored. The adapter device enables carrying out both step A) and step G) of the manual production method of platelet-rich plasma according to the invention preventing the bag from collapsing at the moment of centrifugation, causing a re-mixing of the steps of blood components separated in centrifugation. Consider that the separation container and step L) of the manual production method of platelet-rich plasma according to the invention, by virtue of the Venturi effect, enable uniformly re-suspending the platelets present in the relative sediment, obtaining a homogeneous solution.

Comparative tests between compositions realised according to the invention and realised with means and methods of known type for the production of PRP have demonstrated that the percentage of retrieved platelets and the factor of concentration obtained according to the invention are higher than those relative to the means of known type, enabling higher factors of platelet concentration to be obtained, and totally avoiding the formation of aggregates. In particular, with the single-use kit of the invention it is possible to recuperate a percentage of platelets comprised between 70% and 95%, with a concentration of 6x - 8x and with a reduction of white blood cells of 96% and of red blood cells of 98-100%, with respect to whole blood. This is extremely important to reduce the risk of excessive and damaging inflammatory reactions.

In the present description, as the invention is relative to methods of manual production, each time reference is made to the following terms: "hydraulically connecting", "hydraulically disconnecting", "hydraulic disconnection", "inject", "aspirate" and "collect" the following are to be understood "hydraulically and manually connecting", "hydraulically and manually disconnecting", "manual hydraulic disconnection" "manually inject", "manually aspirate" and "manually collect". Further, although not expressly indicated, when reference is made to "a medical bag", it is to be understood that the medical bag is for the collection of biological liquids. This medical bag preferably has walls made of PVC.

### DESCRIPTION OF THE PREFERRED EMBODIMENTS

The characteristics of the invention will be described.

The scope of the invention is defined by the appended claims.

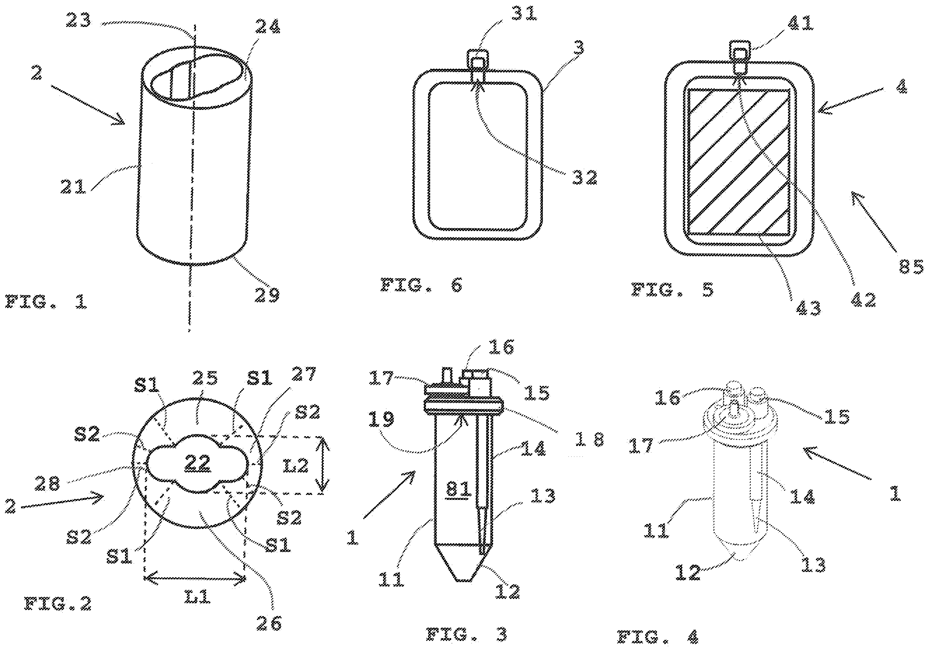

- figure 1 is a perspective view of an adapter device for centrifuge stations of bench centrifuges ;
- figure 2 is a view from above of the adapter device of figure 1.
- figures 3 and 4, are, respectively lateral and perspective views of a separation container according to the invention;
- figure 5 is a schematic front view of a production device of a medication comprising platelet-rich plasma (PRP);
- figure 6 is a schematic front view of a medical bag for the collection of biological liquids utilisable for actuating the method of the invention;
- figures 7-9 are schematic front views that exemplify some steps of the production method of PRP of the invention.

### BRIEF DESCRIPTION OF THE DRAWINGS:

With reference to the figures, reference numeral (1) denotes a separation container according to the invention, reference numeral (2) denotes an adapter device for centrifuge stations of bench centrifuges , and reference numeral (85) denotes a production device of a medication comprising platelet-rich plasma (PRP) .

The separation container (1) is sterilisable and comprises:
- a test tube (11) for a bench centrifuge, the test tube (11) having a relative conical bottom portion (12), a first opening (19) which is opposite the conical bottom portion (12), wherein the test tube (11) internally defines a relative first housing (81);
- a cap (18) which is engageable with the first opening (19) of the test tube (11), the cap (18) having: a first, a second and a third hole which are through-holes; a first and a second needle free connector (15, 16) respectively engaged, in the first and in the second hole in order to close them, wherein, when the cap (18) is engaged with the first opening (19), each of the first and second needle free connectors (15, 16) is arranged in such a way that a relative compression valve (not illustrated) is arranged distally to the test tube (11); a filter (17) engaged in the third hole and having pores of dimensions that are equal to or smaller than 0.4 µm in order to allow only passage of microbiologically filtered air in conjunction with the transfer of a liquid through the first and/or the second needle free connector (15, 16) when the relative compression valve is compressed;
- a tube (14) which originates from the cap (18) at the first needle free connector (15) so that when the cap (18) is engaged with the first opening (19), the tube (14) is arranged internally of the first housing (81) with a relative terminal portion (13), having a relative terminal end, arranged distally of the cap (18) and at the conical bottom portion (12) of the test tube (11), and wherein said terminal portion of the tube (14) is tapered towards the relative terminal in order to obtain a Venturi effect when a liquid is introduced into the test tube (11), through the first needle free connector (15) and the tube (14), to enable re-mixing of a sediment of particles and of a supernatant liquid when contained in the separation container (1).

Starting from the first supernatant liquid obtained from a first centrifugation of whole blood, the separation container is suitable to obtain, by centrifugation, a sediment of platelets and a second supernatant liquid which is constituted by platelet-poor plasma. further, the container (1) enables remixing, by Venturi effect, the platelets of the relative sediment and a part of the second supernatant liquid up to the disappearance of the sediment (see steps L) and M) of the manual production method of platelet-rich plasma according to the invention). It must however be noted that the separation container (1), by virtue of the Venturi effect, can likewise also enable remixing of other types of sediments.

The applicant has experimentally noted that if a container is used for the production of PRP in which, given equal other characteristics with respect to the separation container (1), the tube (14) does not have a tapered portion, or the relative terminal end is not arranged distally of the cap (18) at the conical bottom portion (12) of the test tube (11), or the bottom portion of the test tube (11) is not conical, the resulting PRP does not have the platelet aggregates and is therefore not suitable to be used for the production of the medication. It is obviously that the cap (18) is configured, once engaged with the first opening (19) of the test tube (11), to prevent passage of liquids or air with the outside.

An adapter device (2) for centrifuge stations of bench centrifuges comprises:
- a tubular wall (21) which is externally cylindrical, has a relative axis (23) that is longitudinal and internally defines a second housing (22), which is accessible from a first end (24) of the tubular wall; the second housing (22) having a relative maximum length (L1) that is greater than or equal to a relative maximum width (L2), measured along a transversal plane to the axis (23), wherein the tubular wall (21) has, at a relative first and a relative second longitudinal sector (25, 26), opposite one another with respect to the axis (23), a first thickness (S1) that is uniform and wherein, at a relative third and a relative fourth longitudinal sector (27, 28), which are opposite one another with respect to the axis (23), the longitudinal wall has a relative thickness (S2) that is not uniform and that is smaller than the first thickness (S1), and
- a base wall (not illustrated) which closes the second housing (22) at a second longitudinal end (29) of the tubular wall (21).

The adapter device (2) is insertable in the relative second longitudinal end, in a cylindrical station of a bench centrifuge for, when inserted, enabling housing, in the second housing (22), between the first and second longitudinal sector (25, 26), a test tube (11) for centrifuges having a smaller diameter than the maximum width (L2) and having dimensions such that, during the centrifugation, the test tube (11) remains between the first and second longitudinal sector (25, 26), or enable housing, in the second housing (22), a medical bag (3) for collecting biological liquids, having dimensions such that, at least partially filled, and once housed, it extends from the third and fourth longitudinal sector (27, 28). Considering that the medical bag for collection of biological liquids when empty has a substantially rectangular planar extension, the width and length of the bag will be comparable, with respect to the depth and length (L1) of the second housing (22).

The first and the second longitudinal sector (25, 26) have relative sections, transversal to the axis (23), conformed as arcs of a circular crown and are therefore suitable for retaining a test tube (11) during the centrifugation. The arcs advantageously extend by 3-175°, preferably by 50°.

It is obvious that the adapter device (2) for centrifuge stations of bench centrifuges enables being able to centrifuge a composition, for example blood, while it is contained in the second medical bag (3) for collection of liquids in which it has been collected, by housing the medical bag (3) in the second housing (22) without having to include any decanting of liquids, leading to a possible contamination of the composition. This is particularly important when the composition is blood and it is desired to use it to produce PRP and, subsequently, a medication comprising platelet-rich plasma. It is also obvious that the adapter device (2) alternatively enables housing the test tube (11) for centrifuges, comprised in the separation container (1) of the invention, in the second housing (22), between the first and second longitudinal sector (25, 26). In this way the same centrifuge station and the same adapter device can be used to centrifuge a composition (for example blood) contained in the medical bag for collecting the liquids and a further composition (for example the first supernatant obtainable from the centrifugation of the blood, by centrifuging the blood in the second medical bag) in the test tube (11) for centrifuges of the separation container (1).

It is preferable for the base wall to be perpendicular to the axis (23) and therefore to the tubular wall (21). The second housing (22) preferably has a relative plane of symmetry passing through the axis (23). The production device (85) of a medication comprising platelet-rich plasma is sterile and comprises:
- a first medical bag (4) for collection of biological liquids having a relative first wall and a relative second wall, opposite one another and defining a relative third internal housing, which is accessible from a second opening (42) of the first medical bag (4),
- a third needle free connector (41) arranged in and engaged with the second opening (42), in such a way that a relative compression valve is arranged distally to the third housing;
- a support element (43) (preferably planar) which comprises (or preferably is constituted by): a first support layer made of a bio-resorbable material, preferably porous material; a second layer, in a second bio-resorbable material, arranged on the first layer, wherein the second bio-resorbable material is a polymeric sponge or is a substance selected from a group constituted by: sodium alginate, gelatin, collagen and/or chitosan and combinations thereof and wherein the medical support is contained in the third housing and is arranged with the first layer in contact with the first wall and with the second layer in contact with the second internal wall.

The first support layer thus constitutes a polymeric scaffold and can be advantageously obtainable by 3D printing. This enables controlling the relative thickness and dimension of the relative pores, if porous, which advantageously have dimensions comprised between 0.2 and 2 mm, preferably comprised between 0.5 and 1 mm.

In particular, when porous, the first layer degrades before a non-porous layer and guarantees air permeability, thus facilitating healing of wounds. The grid structure also represents an ideal support for the growth of cells during the regenerating process.

In a preferred embodiment, the first support layer has a thickness comprised between 5 and 500 µm, preferably comprised between 5 and 350 µm and more preferably 150 µm. It is worthy of note that a first layer having a porosity comprised between 0.2 and 2 mm, preferably comprised between 0.5 and 1 mm and a relative thickness of 150 µm, is able to support and/or contain a mixture of the second bio-resorbable material and the PRP which forms by actuating the manual production method of a medication set out in the following. The mixture separating from the first layer is thus avoided. The first bio-resorbable material, preferably porous material, can be: polycaprolactone (PLC), polylactic glycolic acid (PLGA), polyglycolic acid (PGA), and polyvinyl alcohol (PVA), polylactic acid (PLA), NYLON 680, polypropylene (PP), polyethylene (PE), polystyrene, high-impact polystyrene (HIPS), polycarbonate (PC), polyester ether ketone (PEEK), polyetherimide (PEI) and polysulphone (PSU) The preferred embodiments are those in which the first bio-resorbable material is polylactic acid (PLA).

The second layer can have a relative thickness comprised between 1 mm and 10 mm, preferably between 1 mm and 5 mm. The second layer is preferably constituted by the polymeric sponge, preferably made of a substance chosen from the group constituted by: sodium alginate, gelatin, collagen and/or chitosan and combinations thereof. More preferably, this substance is gelatin and is advantageously pork skin gelatin to facilitate absorption of the PRP and the gradual release of growth factors following the application of the medication on the wound. The polymeric sponge advantageously has a relative degree of porosity of 40-60 %, preferably of 45 - 55 %. This is because, in this case, the polymeric sponge absorbs and distributes the PRP more uniformly, enabling the obtaining of a medication with a greater uniformity of distribution of the PRP and starting from a collection of a volume of blood from the patient of a small entity, while maintaining a high concentration of platelets in the final medication.

As the production of PRP requires sterile means, it is particularly advantageous to have a single-use kit for manual production of platelet-rich plasma that enables obtaining a PRP-based medication free of contaminants. The single-use kit comprises:
a second medical bag (3) for collection of biological liquids having a relative second pair of walls, opposite one another and defining a relative fourth internal housing, which is accessible from a third opening (32) of the second medical bag (3), a fourth needle free connector (31) arranged in and engaged with the third opening (32) in such a way that a relative compression valve is arranged distally to the fourth housing;
- a separation container (1) according to the invention, sterile; and
- a closed and sterile hydraulic circuit (5) comprising: a first hydraulic conduit (51) having a first inlet (52), hydraulically connectable to a needle free connector in order to compress the relative compression valve, and a first outlet, and being flowable through by a liquid only in a first direction (V1) from the first inlet (52) to the first outlet; a hydraulic bifurcation arranged downstream of the first hydraulic conduit (51), with respect to the first direction (V1), said bifurcation comprises a second hydraulic conduit (53) which can be flowed through by a fluid only in a single second direction (V2), concordant with the first direction (V1), towards a relative second outlet (54) hydraulically connectable to a needle free connector to compress the relative compression valve thereof; and a third hydraulic portion (56) which can be flowed through in both relative directions (V3, V4), from or towards a relative third outlet; wherein the first inlet (52) comprises a relative connector (57) engageable with the fourth needle free connector (31) which, when engaged, compresses the relative compression valve thereof to hydraulically connect the second medical bag (3) with the first hydraulic circuit;
- a first manual collecting and injecting medical device (6), which is sterile.

The first inlet (52) can comprise a luer-lock connector (57), i.e. a connector that has a relative threading engageable with a needle free connector to compress the relative compression valve. The first hydraulic conduit (51) advantageously comprises a first single-acting hydraulic valve (59) arranged upstream of the bifurcation and contiguous to the bifurcation. The second hydraulic conduit (53) advantageously comprises a first single-acting hydraulic valve (50) arranged upstream of the second outlet (54) and contiguous to the second outlet (54).

According to an embodiment, not illustrated, the single-use kit preferably further comprises: a second manual collecting and injecting medical device, which is sterile; a third manual collecting and injecting medical device, which is sterile and filled with an anti-coagulant agent; and a female-female hydraulic connector for mutually connecting the second and third manual collecting and injecting medical device, enabling introduction of the anti-coagulant agent into the second collecting and injecting medical device the anti-coagulant agent.

A preferred embodiment of the invention relates to a re-utilisable centrifugation kit for manual production of platelet-rich plasma, comprising:
- a pair of adapter devices (2) as above disclosed, for centrifuge stations of bench centrifuges, identical to one another;
- a third medical bag for collection of biological liquids of dimensions such that, when at least partially filled, and once housed, it extends from the third and fourth longitudinal sector of the second housing (22) of one of the adapter devices of the pair of adapter devices (2);
- a further separation container (1) according to the invention.

The third medical bag, once having been appropriately filled and inserted in one of the two adapter devices (2) of the reutilisable kit, is destined to be used as a counterweight to the second medical bag (3) during step A) of the manual production method of platelet-rich plasma according to the invention and to the foregoing. Obviously the two adapter devices (2) must be inserted in two different centrifuge stations opposite one another across the rotation axis and the second medical bag (3), containing the blood, will be inserted in the second housing (22) of one of the two adapter devices, and the third medical bag, filled with a suitable quantity of liquid for counterbalancing the quantity of blood present in the second medical bag (3), will be inserted in the second housing (22) of the other of the two adapter devices. While the further separation container (1) according to the invention, once having been appropriately filled and inserted in one of the two adapter devices (2) of the reutilisable kit (at the second housing 22), is destined to be a counterweight to the separation container (1) containing the first supernatant liquid (34) during step G) of the method in which the first separation container (1) is in the second housing (22) of the remaining adapter device of the re-utilisable centrifugation kit. This enables carrying out step A) and step G) of the manual production method of platelet-rich plasma according to the invention. Note that both the second and the third medical bag (3), even though at least partially filled with a liquid and inserted directly in a cylindrical station of a bench centrifuge, would not be suitably retained by the cylindrical station. This does not allow, in relation to the second medical bag (3), for a suitable separation of the red blood cells by centrifugation.

In a relative preferred embodiment, the invention relates to a single-use kit for manual production of a medication. It comprises a production device (85) of a medication as above desclosed; a single-use kit for manual production of platelet-rich plasma according to the invention, and a gelling agent, preferably a calcium salt such as calcium gluconate or calcium chloride or thrombin, most preferably calcium gluconate.

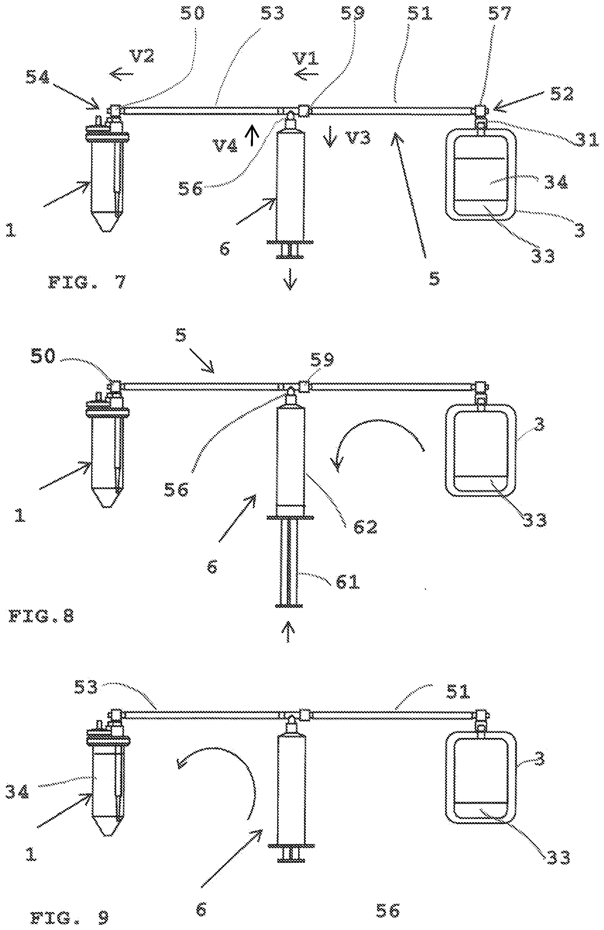

A manual production method of platelet-rich plasma comprises following steps:
A) centrifuging blood collected from a patient, and added-to with an anti-coagulant agent, while it is contained in the second medical bag (3) for collection of biological liquids so at to obtain, on the bottom of the second medical bag (3), a sediment (33) of red blood cells and a remaining first supernatant liquid (34): B) predisposing a closed and sterile hydraulic pathway comprising: a first hydraulic portion having a first inlet (52) that is hydraulically connectable to a needle free connector to compress the relative compression valve thereof and a first outlet and being flowable through by a liquid only in a first direction from the inlet to the outlet; a hydraulic bifurcation arranged downstream of the first hydraulic portion, with respect to the first direction, the bifurcation comprising a second hydraulic portion which can be flowed through by a fluid only in a second direction, concordant with the first direction, towards a relative second outlet (54) hydraulically connectable to a needle free connector to compress the relative compression valve thereof; and a third hydraulic portion which can be flowed through in both directions, from or towards a relative third outlet: C) hydraulically connecting: the first inlet (52) to the second medical bag (3) (obviously by means of the fourth needle free connector (31) thereof by compressing the relative compression valve); the second outlet (54) to a separation container (1) according to the invention and sterilised (preferably by means of the second needle free connector (16) thereof by compressing the relative compression valve); and the third outlet to the first manual collecting and injecting medical device (6) of a liquid;
D) aspirating the first supernatant liquid (34) from the second medical bag (3), via the first manual collecting and injecting medical device (6) while the first manual collecting and injecting medical device (6) is connected to the third outlet for storage thereof in the first manual collecting and injecting medical device (6) (see figure 8, where the curved arrow indicates the pathway of the first supernatant liquid (34));
E) injecting into the closed hydraulic pathway, via the first manual collecting and injecting medical device (6) connected to the third outlet, the first supernatant liquid (34) stored there in order to transfer the first supernatant liquid (34) into the separation container (1) (see figure 9, where the curved arrow indicates the pathway of the first supernatant liquid (34));
F) hydraulically disconnecting the separation container (1) from the second outlet (54);
G) centrifuging the first supernatant liquid (34) while it is contained in the separation container (1) obtaining a sediment of platelets and a second supernatant liquid which is constituted by platelet-poor plasma, or PPP;
H) hydraulically connecting a fourth manual collecting and injecting medical device a liquid to the second needle free connector (16) in order to compress the relative compression valve; overturning the separation container (1) and collecting the second supernatant liquid, via the fourth manual collecting and injecting medical device for, and storing the second supernatant liquid therein, newly overturning the separation container (1) and injecting, via the fourth manual collecting and injecting medical device, a predetermined part of the stored second supernatant liquid, obtaining a third liquid contained in the separation container;
I) disconnecting the fourth collecting and injecting medical device from the second needle free connector (16) and hydraulically connecting, to the first needle free connector (15) of the separation container (1), a fifth manual collecting and injecting medical device;
L) via the fifth manual collecting and injecting medical device, connected to the first needle free connector (15), collecting the third liquid from the separation container (1) and re-injecting the third liquid, obtaining a Venturi effect in order to remix the platelets of the sediment of platelets and the third liquid;
M) reiterating step L) up until the disappearance of the sediment and the obtaining of a platelet-rich plasma, wherein the relative platelets are uniformly suspended, and storing the plasma in the fifth manual collecting and injecting medical device.

It is to be considered that step A) of the manual production method of PRP mentioned in the foregoing can be carried out following insertion of the second medical bag 3 in an adapter device 2 for centrifuge stations of bench centrifuges as above disclosed and following insertion of the adapter device (2) in a centrifuge station of a bench centrifuge. Further, the step G) can be obtained following insertion in the separation container (1) in the second housing (22) of the adapter device (2) between the first and second longitudinal sector (25, 26), and following insertion of the adapter device (2) for centrifuge stations in the centrifuge station. Note that in the second housing (22) a test tube for centrifuges can be housed, and therefore also the test tube relative to the separation container (1).

Additionally, it is particularly remarkable that the closed and sterile hydraulic circuit contained in the single-use kit for manual production of PRP enables carrying out step B) of the manual production method of platelet-rich plasma. The hydraulic circuit (5) comprises characteristics corresponding to those mentioned in step B). Therefore, the single-use kit for manual production of PRP enables carrying out steps B), C) D). Further, the re-utilisable centrifugation kit enables carrying out step A) and G) of the method, respectively using the third medical bag and the separation container (1) contained therein as counterweights. Note that in step H), differently to the numerous other sediments obtained by centrifugation, given the nature of the sediment of platelets, during the overturning of the separation container (1) it does not detach from the bottom thereof. The fact that the second supernatant liquid is collected in the overturned position means that it can all be collected. The tapered terminal portion (13) of the tube (14) enables obtaining, in step L), the Venturi effect and therefore effectively homogeneously re-suspending the platelets without having to open the separation container (1).

The fourth manual collecting and injecting medical device can be the same first collecting and injecting medical device (6) used previously, if it is hydraulically connectable to a needle-free connector. The fifth manual collecting and injecting medical device can be the same fourth manual collecting and injecting medical device, but is preferably different and has a smaller capacity to increase the Venturi effect.

Following is disclosed a manual production method of a medication comprising platelet-rich plasma. The said manual production method comprises the following steps:
N) predisposing platelet-rich plasma;
O) manually injecting the platelet-rich plasma, optionally added-to by a coagulating agent, into the third housing of a production device (85) of a medication as above disclosed :
   P) homogeneously distributing the platelet-rich plasma in the support element (43) of the production device (85) of a medication, preferably by compression of the support element (43), and of the platelet-rich plasma injected, contained in the third housing between the first wall and the second wall.

Step P), relating to distribution, enables obtaining a medication in which the PRP is also homogeneously distributed on large surfaces with small volumes of PRP, to cover large lesions.

It is advantageous for step N) of predisposing the platelet-rich plasma to comprise predisposing the platelet-rich plasma obtained according to the manual production method of platelet-rich plasma according to the invention. In this case the use is included of the fifth medical device for collection and step O) of injecting the platelet-rich plasma comprises following sub-steps:
O1) hydraulically connecting the fifth manual collecting and injecting medical device to the third needle free connector (41) of the production device (85) of a medication; and
O2) injecting the platelet-rich plasma into the third housing, via the fifth manual collecting and injecting medical device connected to the third needle free connector (41).

It is further of considerable importance that the manual production method of the medication enables directly obtaining a package (not illustrated) comprising the medication enabling the avoiding of further packaging steps when it is not expected to have to use the medication produced immediately. The package comprises a packaging and a medication containing in the packaging, where the medication comprises platelet-rich plasma, a support element (43) which in time comprises: a first support layer made of a first bio-resorbable and porous material; a second layer, in a second bio-resorbable material, arranged on the first layer, wherein the second bio-resorbable material is a polymeric sponge or is a substance selected from a group constituted by: sodium alginate, gelatin, collagen and/or chitosan and combinations thereof, wherein the platelet-rich plasma is dispersed in the second bio-resorbable material, and wherein the second layer is arranged on the first layer. The package is characterised in that the packaging is a medical bag, preferably made of PVC, for collection of biological liquids having a relative first wall and a relative second wall, opposite one another and defining a relative internal housing; in that the first layer is in contact with the first wall; in that the second layer is in contact with the second internal wall, and in that the housing is accessible from an opening of the medical bag in which a needle free connector is arranged, in such a way that a relative compression valve is arranged distally to the housing of the medical bag. The preferred embodiments described in the foregoing in relation to the production device (85) and to the support element (43), to the first and second layer, to the first and a second bio-resorbable material are applied also to the package.

Purely by way of example some dimensions of some elements described herein are given. The medical bags for collection of biological liquids can have a relative housing of 70 ml and can be filled up to 60 ml to be introduced in the adapter device (2) as above disclosed and subject to centrifugation.

Consequently it is preferable for the test tube (11) for a centrifuge of the separation container (1) according to the invention has a relative length comprised between 40 and 160 mm, more preferably comprised between 60 and 95 mm; and for the relative first opening (19) to have a diameter comprised between 20 and 60 mm, more preferably comprised between 28 and 50 mm. The tube (14) can instead have a relative length comprised between 37 and 157 mm, more preferably comprised between 57 and 92 mm, a relative maximum diameter comprised between 1 and 6 mm, more preferably comprised between 3 and 4 mm, with a minimum diameter of the tapered terminal portion, i.e. the diameter of the terminal hole of the tube (14) comprised between 0.1 and 3 and preferably between 0.5 and 1.5 mm. The distance of the terminal end of the tube (14), with respect to the conical bottom portion (12), is advantageously comprised between 0.5 and 38 mm, more preferably comprised between 1 and 16 mm.

## Claims

1. A separation container (1) for production of platelet-rich plasma from blood, which is sterilisable and comprising:
- a test tube (11) for a bench centrifuge, the test tube (11) having a relative conical bottom portion (12), a first opening (19) which is opposite the conical bottom portion (12), wherein the test tube (11) internally defines a relative first housing (81);
- a cap (18) which is engageable with the first opening (19) of the test tube (11), the cap (18) having: a first, a second and a third hole which are through-holes;
- a tube (14) which originates from the cap (18) at the first needle free connector (15) so that, when the cap (18) is engaged with the first opening (19), the tube (14) is arranged internally of the first housing (81) with a relative terminal portion (13) having a relative terminal end, arranged distally of the cap (18) and at the conical bottom portion (12) of the test tube (11), **characterized in that** the separation container (1) further comprises:
a first and a second needle free connector (15, 16) respectively engaged, in the first and in the second hole in order to close them, wherein, when the cap (18) is engaged with the first opening (19), each of the first and second needle free connectors (15, 16) is arranged in such a way that a relative compression valve is arranged distally to the test tube (11);
a filter (17) engaged in the third hole and having pores of dimensions that are equal to or smaller than 0.4 µm in order to allow only passage of microbiologically filtered air in conjunction with the transfer of a liquid through the first and/or the second needle free connector (15, 16) when the relative compression valve is compressed;
- the separation container (1) being further **characterized in that** said terminal portion of the tube (14) is tapered towards the relative terminal in order to obtain a Venturi effect when a liquid is introduced into the test tube (11), through the first needle free connector (15) and the tube (14), to enable re-mixing of a sediment of particles and of a supernatant liquid when contained in the separation container (1).

2. A single-use kit for manual production of platelet-rich plasma, comprising:
- a separation container (1) according to claim 1, sterile;
- a first medical bag (3) for collection of biological liquids having a relative pair of walls, opposite one another and defining a relative second internal housing, which is accessible from a third opening (32) of the first medical bag (3), a fourth needle free connector (31) arranged in and engaged with the third opening (32) in such a way that a relative compression valve is arranged distally to the second housing;
- a closed and sterile hydraulic circuit (5) comprising: a first hydraulic conduit (51) having a first inlet (52), hydraulically connectable to a needle free connector in order to compress the relative compression valve, and a first outlet, wherein the first hydraulic conduit (51) can be flowed through by a liquid only in a first direction (V1) from the first inlet (52) to the first outlet; a hydraulic bifurcation arranged downstream of the first hydraulic conduit (51), with respect to the first direction (V1), said bifurcation comprising a second hydraulic conduit (53) which can be flowed through by a fluid only in a single second direction (V2), concordant with the first direction (V1), towards a relative second outlet (54) hydraulically connectable to a needle free connector to compress the relative compression valve thereof; and a third hydraulic portion (56) which can be flowed through in both relative directions (V3, V4), from or towards a relative third outlet; wherein the first inlet (52) comprises a relative connector (57) engageable with the fourth needle free connector (31) which, when engaged, compresses the relative compression valve to hydraulically connect the first medical bag (3) with the first hydraulic circuit; and
- a first manual collecting and injecting medical device (6), which is sterile.

3. A single-use kit for manual production of platelet-rich plasma according to claim 2, further comprising:
- a second manual collecting and injecting medical device, which is sterile;
- a third manual collecting and injecting medical device, which is sterile and filled with an anti-coagulant agent and
- a female-female hydraulic connector for mutually connecting the second and third manual collecting and injecting medical device, enabling introduction of the anti-coagulant agent into the second collecting and injecting medical device.

4. A re-utilisable centrifugation kit for manual production of platelet-rich plasma, comprising:
- a separation container (1) according to claim 1;
- a pair of adapter devices (2) for centrifuge stations of bench centrifuges identical to one another wherein each of said adapter device comprising: a tubular wall (21) which is externally cylindrical; the adapter device (2) has a relative axis (23) that is longitudinal and internally defines a third housing (22), which is accessible from a first end (24) of the tubular wall; the third housing (22) having a relative maximum length (L1) that is greater than or equal to a relative maximum width (L2), measured along a transversal plane to the axis (23), wherein the tubular wall (21) has, at a relative first and a relative second longitudinal sector (25, 26), opposite one another with respect to the axis (23), a first thickness (S1) that is uniform and wherein, at a relative third and a relative fourth longitudinal sector (27, 28), which are opposite one another with respect to the axis (23), the longitudinal wall has a relative thickness (S2) that is not uniform and that is smaller than the first thickness (S1), and a base wall which closes the third housing (22) at a second longitudinal end (29) of the tubular wall (21); the adapter device (2) being insertable in the relative second longitudinal end, in a cylindrical station of a bench centrifuge for, when inserted, enabling housing, in the third housing (22), between the first and second longitudinal sector (25, 26), a test tube (11) for centrifuges having a smaller diameter than the maximum width (L2) and having dimensions such that, during the centrifugation, the test tube (11) remains between the first and second longitudinal sector (25, 26), or enabling housing, in the third housing (22), a first medical bag (3) for collecting liquids, having dimensions such that, when at least partially filled, and once housed, it extends from the third and fourth longitudinal sector (27, 28);
- a second further medical bag for collection of biological liquids of dimensions such that, when at least partially filled, and once housed, the second medical bag extends from the third to the fourth longitudinal sector of the third housing (22) of one of the adapter devices of the pair of adapter devices.

5. A single-use kit for manual production of a medication, comprising: a single-use kit for manual production of platelet-rich plasma according to claims 2 or 3; a production device (85) of a medication comprising platelet-rich plasma, the production device (85) being sterile and comprising:
- a fourth medical bag (4) for collection of biological liquids having a relative first wall and a relative second wall, opposite one another and defining a relative fourth internal housing, which is accessible from a second opening (42) of the fourth medical bag (4),
- a third needle free connector (41) arranged in and engaged with the second opening (42), in such a way that a relative compression valve is arranged distally to the fourth housing;
a support element (43) which comprises: a first support layer made of a bio-resorbable material, preferably porous; a second layer, made of a second bio-resorbable material, arranged on the first layer, wherein the second bio-resorbable material is a polymeric sponge or is a substance selected from a group constituted by: sodium alginate, gelatin, collagen and/or chitosan and combinations thereof and wherein the medical support is contained in the fourth housing and is arranged with the first layer in contact with the first wall and with the second layer in contact with the second internal wall and, optionally, a gelling agent.

6. A manual production method of platelet-rich plasma, the method being **characterized by** comprising following steps:
A) centrifuging blood collected from a patient, and added-to with an anti-coagulant agent, while it is contained in a medical bag (3) for collection of biological liquids so at to obtain, on the bottom of the medical bag (3), a sediment (33) of red blood cells and a remaining first supernatant liquid (34):
B) predisposing a closed and sterile hydraulic pathway comprising: a first hydraulic portion having a first inlet (52) that is hydraulically connectable to a needle free connector to compress the relative compression valve thereof and a first outlet wherein the first hydraulic conduit (51) can be flowed through by a liquid only in a first direction from the inlet to the outlet; a hydraulic bifurcation arranged downstream of the first hydraulic portion, with respect to the first direction, the bifurcation comprising a second hydraulic portion which can be flowed through by a fluid only in a single second direction, concordant with the first direction, towards a relative second outlet (54) hydraulically connectable to a needle free connector, in order to compress the relative compression valve thereof; and a third hydraulic portion which can be flowed through in both directions, from or towards a relative third outlet:
C) hydraulically connecting: the first inlet (52) to the medical bag (3); the second outlet (54) to a separation container (1) according to claim 1 and sterilised; and the third outlet to the first manual collecting and injecting medical device (6) of a liquid;
D) aspirating the first supernatant liquid (34) from the medical bag (3), via the first manual collecting and injecting medical device (6) while the first manual collecting and injecting medical device (6) is connected to the third outlet for storage thereof in the first manual collecting and injecting medical device (6);
E) injecting into the closed hydraulic pathway, via the first manual collecting and injecting medical device (6) connected to the third outlet, the first supernatant liquid (34) stored there in order to transfer the first supernatant liquid (34) into the separation container (1);
F) hydraulically disconnecting the separation container (1) from the second outlet (54);
G) centrifuging the first supernatant liquid (34) while it is contained in the separation container (1), obtaining a sediment of platelets and a second supernatant liquid which is constituted by platelet-poor plasma;
H) hydraulically connecting a second manual collecting and injecting medical device of a liquid to the second needle free connector (16) in order to compress the relative compression valve; overturning the separation container (1) and collecting the second supernatant liquid, via the second manual collecting and injecting medical device, and storing the second supernatant liquid therein, newly overturning the separation container (1) and injecting, via the second manual collecting and injecting medical device, a predetermined part of the stored second supernatant liquid, obtaining a third liquid contained in the separation container (1);
I) disconnecting the second collecting and injecting medical device from the second needle free connector (16) and hydraulically connecting, to the first needle free connector (15) of the separation container (1) a third manual collecting and injecting medical device;
L) via the third manual collecting and injecting medical device, connected to the first needle free connector (15), collecting the third liquid from the separation container (1) and re-injecting the third liquid, obtaining a Venturi effect in order to remix the platelets of the sediment of platelets and the third liquid;
M) reiterating step L) up until the disappearance of the sediment and the obtaining of a platelet-rich plasma, wherein the relative platelets are uniformly suspended, and storing the plasma in the third manual collecting and injecting medical device.

## Patentansprüche

1. Separationsbehälter (1) zur Herstellung von thrombozytenreichem Plasma (auch "plättchenreiches Plasma" oder "PRP" genannt) aus Blut, der sterilisierbar ist und Folgendes umfasst:
- ein Probenröhrchen (11) für eine Tischzentrifuge, wobei das Probenröhrchen (11) einen entsprechenden konischen Bodenabschnitt (12) und eine erste Öffnung (19), die dem konischen Bodenabschnitt (12) gegenüberliegt, aufweist, wobei das Probenröhrchen (11) in seinem Inneren eine entsprechende erste Aufnahme (81) definiert,
- eine Kappe (18), die mit der ersten Öffnung (19) des Probenröhrchens (11) in Eingriff treten kann, wobei die Kappe (18) aufweist: ein erstes, ein zweites und ein drittes Loch, welche Durchgangslöcher sind,
- ein Röhrchen (14), das von der Kappe (18) ausgehend an einem ersten nadellosen Konnektor (15) seinen Ursprung nimmt, so dass, wenn die Kappe (18) mit der ersten Öffnung (19) in Eingriff steht, das Röhrchen (14) innerhalb der ersten Aufnahme (81) angeordnet ist, wobei ein entsprechender Endabschnitt (13) ein entsprechendes Abschlussende aufweist, das distal von der Kappe (18) und an dem konischen Bodenabschnitt (12) des Probenröhrchens (11) angeordnet ist,
**dadurch gekennzeichnet, dass** der Separationsbehälter (1) ferner umfasst:
zusätzlich zu dem ersten nadellosen Konnektor (15) einen zweiten nadellosen Konnektor (16), die jeweils in dem ersten beziehungsweise in dem zweiten Loch eingelassen sind, um diese zu verschließen, wobei, wenn die Kappe (18) mit der ersten Öffnung (19) in Eingriff steht, sowohl der erste als auch der zweite nadellose Konnektor (15, 16) derart angeordnet sind, dass ein entsprechendes Kompressionsventil distal von dem Probenröhrchen (11) angeordnet ist;
einen Filter (17), der in das dritte Loch eingesetzt ist und Poren mit Abmessungen aufweist, die gleich oder kleiner als 0,4 µm sind, um nur den Durchlass von mikrobiologisch gefilterter Luft in Verbindung mit dem Transfer einer Flüssigkeit durch den ersten und/oder den zweiten nadellosen Konnektor (15, 16) zu erlauben, wenn das relative Kompressionsventil komprimiert ist;
- wobei der Separationsbehälter (1) ferner **dadurch gekennzeichnet ist, dass** sich der Endabschnitt des Röhrchens (14) zu dem entsprechenden Ende hin verjüngt, um einen Venturi-Effekt zu erhalten, wenn eine Flüssigkeit, durch den ersten nadellosen Konnektor (15) und das Röhrchen (14), in das Probenröhrchen (11) eingegeben wird, um das erneute Vermischen eines Sediments von Partikeln und einer überstehenden Flüssigkeit zu ermöglichen, wenn diese in dem Separationsbehälter (1) enthalten sind.

2. Kit für den Einmalgebrauch zur manuellen Herstellung von thrombozytenreichem Plasma, umfassend:
- einen Separationsbehälter (1) nach Anspruch 1, der steril ist;
- einen ersten medizinischen Beutel (3) zur Entnahme von biologischen Flüssigkeiten, der ein entsprechendes Paar Wände aufweist, die einander gegenüberliegen und eine entsprechende zweite innere Aufnahme definieren, die über eine dritte Öffnung (32) des ersten medizinischen Beutels (3) zugänglich ist, wobei ein vierter nadelloser Konnektor (31) derart in der dritten Öffnung (32) angeordnet und eingelassen ist, dass ein entsprechendes Kompressionsventil distal zu der zweiten Aufnahme angeordnet ist;
- einen geschlossenen und sterilen Hydraulikkreislauf (5), umfassend:
eine erste hydraulische Leitung (51), die einen ersten Einlass (52) aufweist, der hydraulisch mit einem nadellosen Konnektor verbindbar ist, um das entsprechende Kompressionsventil zu komprimieren, und einen ersten Auslass aufweist, wobei die erste hydraulische Leitung (51) nur in einer ersten Richtung (V1) von dem ersten Einlass (52) zu dem ersten Auslass hin von einer Flüssigkeit durchströmt werden kann, eine hydraulische Verzweigung, die stromabwärts von der ersten hydraulischen Leitung (51), in Bezug auf die erste Richtung (V1) betrachtet, angeordnet ist, wobei die Verzweigung eine zweite hydraulische Leitung (53) umfasst, die nur in einer einzigen zweiten Richtung (V2), die mit der ersten Richtung (V1) übereinstimmt, zu einem entsprechenden zweiten Auslass (54) hin von einer Flüssigkeit durchströmt werden kann, der hydraulisch mit einem nadellosen Konnektor verbindbar ist, um dessen entsprechendes Kompressionsventil zu komprimieren; und eine dritte hydraulische Leitung (56), die in beiden entsprechenden Richtungen (V3, V4) durchströmt werden kann, von oder zu einem entsprechenden dritten Auslass; wobei der erste Einlass (52) einen entsprechenden Konnektor (57) umfasst, der mit dem vierten nadellosen Konnektor (31) ein Eingriff treten kann und der, wenn er in Eingriff steht, das entsprechende Kompressionsventil komprimiert, um eine hydraulische Verbindung zwischen dem ersten medizinischen Beutel (3) und dem ersten Hydraulikkreislauf herzustellen;
- eine erste manuelle medizinische Entnahme- und Injektionsvorrichtung (6), die steril ist.

3. Kit für den Einmalgebrauch zur manuellen Herstellung von thrombozytenreichem Plasma nach Anspruch 2, ferner umfassend:
- eine zweite manuelle medizinische Entnahme- und Injektionsvorrichtung, die steril ist;
- eine dritte manuelle medizinische Entnahme- und Injektionsvorrichtung, die steril ist und mit einem blutgerinnungshemmenden Mittel gefüllt ist, und
- einen weiblich/weiblich hydraulischen Konnektor zum Verbinden der zweiten und dritten manuellen medizinischen Entnahme- und Injektionsvorrichtung miteinander, um das Eingeben des blutgerinnungshemmenden Mittels in die zweite medizinische Entnahme- und Injektionsvorrichtung zu ermöglichen.

4. Wiederverwendbares Zentrifugations-Kit zur manuellen Herstellung von thrombozytenreichem Plasma, umfassend:
- einen Separationsbehälter (1) nach Anspruch 1;
- ein Paar Adaptervorrichtungen (2) für Zentrifugenstellplätze von Tischzentrifugen, die miteinander identisch sind, wobei jede der Adaptervorrichtungen umfasst: eine rohrförmige Wand (21), die außen zylindrisch ist; wobei jede Adaptervorrichtung (2) eine entsprechende längsgerichtete Achse (23) aufweist und in ihrem Inneren eine dritte Aufnahme (22) definiert, die von einem ersten Ende (24) der rohrförmigen Wand aus zugänglich ist; wobei die dritte Aufnahme (22) eine entsprechende maximale Länge (L1) aufweist, die größer oder gleich einer entsprechenden maximalen Breite (L2) ist, gemessen entlang einer zur Achse (23) quergerichteten Ebene, wobei die rohrförmige Wand (21), an einem entsprechenden ersten und einem entsprechenden zweiten Längssektor (25, 26), die bezogen auf die Achse (23) einander gegenüberliegen, eine erste Dicke (S1) aufweist, die gleichförmig ist, und wobei, an einem entsprechenden dritten und einem entsprechenden vierten Längssektor (27, 28), die bezogen auf die Achse (23) einander gegenüberliegen, die Längswand eine entsprechende Dicke (S2) aufweist, die nicht gleichförmig ist und die kleiner als die erste Dicke (S 1) ist, und eine Basiswand, welche die dritte Aufnahme (22) an einem zweiten Längsende (29) der rohrförmigen Wand (21) abschließt; wobei jede Adaptervorrichtung (2) über das entsprechende zweite Längsende in einen zylindrischen Stellplatz einer Tischzentrifuge einsetzbar ist, um, wenn sie eingesetzt ist, zu ermöglichen, dass in der dritten Aufnahme (22), zwischen dem ersten und dem zweiten Längssektor (25, 26), ein Probenröhrchen (11) für Zentrifugen aufgenommen wird, dessen Durchmesser kleiner ist als die maximale Breite (L2) und der solche Abmessungen aufweist, dass, während des Zentrifugierens, das Probenröhrchen (11) zwischen dem ersten und dem zweiten Längssektor (25, 26) verbleibt, oder um zu ermöglichen, dass in der dritten Aufnahme (22) ein erster medizinischer Beutel (3) zur Entnahme von Flüssigkeiten aufgenommen wird, der solche Abmessungen aufweist, dass er sich, wenn er zumindest teilweise gefüllt ist und sich in der Aufnahme befindet, von dem dritten zu dem vierten Längssektor (27, 28) erstreckt;
- einen zweiten, weiteren medizinischen Beutel zur Entnahme von biologischen Flüssigkeiten, der solche Abmessungen aufweist, dass sich der zweite medizinische Beutel, wenn er zumindest teilweise gefüllt ist und sich in der Aufnahme befindet, von dem dritten zu dem vierten Längssektor der dritten Aufnahme (22) einer der Adaptervorrichtungen des Paars von Adaptervorrichtungen erstreckt.

5. Kit für den Einmalgebrauch zur manuellen Herstellung eines Arzneimittels, umfassend: ein Kit für den Einmalgebrauch zur manuellen Herstellung von thrombozytenreichem Plasma nach den Ansprüchen 2 oder 3; eine Produktionsvorrichtung (85) zur Herstellung eines Arzneimittels, das thrombozytenreiches Plasma enthält, wobei die Produktionsvorrichtung (85) steril ist und umfasst:
- einen vierten medizinischen Beutel (4) zur Entnahme von biologischen Flüssigkeiten, der eine entsprechende erste Wand und eine entsprechende zweite Wand aufweist, die einander gegenüberliegen und eine entsprechende vierte innere Aufnahme definieren, die über eine zweite Öffnung (42) des vierten medizinischen Beutels (4) zugänglich ist,
- einen dritten nadellosen Konnektor (41), der derart in der zweiten Öffnung (42) angeordnet und eingelassen ist, dass ein entsprechendes Kompressionsventil distal zu der vierten Aufnahme angeordnet ist;
- ein Trägerelement (43), umfassend: eine erste Trägerschicht aus einem bioresorbierbaren, vorzugsweise porösen, Material; eine zweite Schicht aus einem zweiten bioresorbierbaren Material, die auf der ersten Schicht angeordnet ist, wobei die zweite bioresorbierbare Material ein polymerer Schwamm ist oder eine Substanz, die ausgewählt ist aus einer Gruppe bestehend aus: Natriumalginat, Gelatine, Collagen und/oder Chitosan und Kombinationen derselben, und wobei der medizinische Träger in der vierten Aufnahme enthalten ist und so angeordnet ist, dass die erste Schicht in Kontakt mit der ersten Wand ist und die zweite Schicht in Kontakt mit der zweiten Innenwand ist, und, optional, einen Gelbildner.

6. Manuelles Verfahren zur Herstellung von thrombozytenreichem Plasma, wobei das Verfahren **dadurch gekennzeichnet ist, dass** es folgende Schritte beinhaltet:
A) Zentrifugieren von Blut, das einem Patienten entnommen wurde und dem ein blutgerinnungshemmendes Mittel zugesetzt wurde, während es in einem medizinischen Beutel (3) zur Entnahme von biologischen Flüssigkeiten enthalten ist, um, am Boden des medizinischen Beutels (3), ein Sediment (33) von Erythrozyten und eine verbleibende erste überstehende Flüssigkeit (34) zu erhalten;
B) Bereitstellen eines geschlossenen und sterilen Hydraulikkreislaufs, umfassend: eine erste hydraulische Leitung, die einen ersten Einlass (52) aufweist, der hydraulisch mit einem nadellosen Konnektor verbindbar ist, um dessen entsprechendes Kompressionsventil zu komprimieren, und einen ersten Auslass aufweist, wobei die erste hydraulische Leitung (51) nur in einer ersten Richtung von dem Einlass zu dem Auslass hin von einer Flüssigkeit durchströmt werden kann, eine hydraulische Verzweigung, die stromabwärts von der ersten hydraulischen Leitung, in Bezug auf die erste Richtung betrachtet, angeordnet ist, wobei die Verzweigung eine zweite hydraulische Leitung umfasst, die nur in einer einzigen zweiten Richtung, die mit der ersten Richtung übereinstimmt, zu einem entsprechenden zweiten Auslass (54) hin von einer Flüssigkeit durchströmt werden kann, der hydraulisch mit einem nadellosen Konnektor verbindbar ist, um dessen entsprechendes Kompressionsventil zu komprimieren; und eine dritte hydraulische Leitung, die in beiden entsprechenden Richtungen durchströmt werden kann, von oder zu einem entsprechenden dritten Auslass;
C) hydraulisches Verbinden: des ersten Einlasses (52) mit dem medizinischen Beutel (3); des zweiten Auslasses (54) mit einem Separationsbehälter (1) nach Anspruch 1, der sterilisiert ist; und des dritten Auslasses mit der ersten manuellen medizinischen Entnahme- und Injektionsvorrichtung (6) einer Flüssigkeit;
D) Absaugen der ersten überstehenden Flüssigkeit (34) aus dem medizinischen Beutel (3), über die erste manuelle medizinische Entnahme- und Injektionsvorrichtung (6), während die erste manuelle medizinische Entnahme- und Injektionsvorrichtung (6) mit dem dritten Auslass verbunden ist, um diese Flüssigkeit in der ersten manuellen medizinischen Entnahme- und Injektionsvorrichtung (6) aufzubewahren;
E) Injizieren, in den ersten geschlossenen Hydraulikkreislauf, über die erste manuelle medizinische Entnahme- und Injektionsvorrichtung (6), die mit dem dritten Auslass verbunden ist, der ersten überstehenden Flüssigkeit (34), die darin aufbewahrt ist, um die erste überstehende Flüssigkeit (34) in den Separationsbehälter (1) zu transferieren;
F) hydraulisches Trennen des Separationsbehälters (1) von dem zweiten Auslass (54);
G) Zentrifugieren der ersten überstehenden Flüssigkeit (34), während diese in dem Separationsbehälter (1) enthalten ist, zum Erhalten eines Sediments von Thrombozyten und einer zweiten überstehenden Flüssigkeit, die aus thrombozytenarmem Plasma besteht;
H) hydraulisches Verbinden einer zweiten manuellen medizinischen Entnahme- und Injektionsvorrichtung einer Flüssigkeit mit dem zweiten nadellosen Konnektor (16), um das entsprechende Kompressionsventil zu komprimieren; Umdrehen des Separationsbehälters (1) und Entnehmen der zweiten überstehenden Flüssigkeit, über die zweite manuelle medizinische Entnahme- und Injektionsvorrichtung, und Aufbewahren der zweiten überstehenden Flüssigkeit darin, erneutes Umdrehen des Separationsbehälters (1) und Injizieren, über die zweite manuelle medizinische Entnahme- und Injektionsvorrichtung, eines vorbestimmten Teils der aufbewahrten zweiten überstehenden Flüssigkeit, so dass eine dritte Flüssigkeit erhalten wird, die in dem Separationsbehälter (1) enthalten ist;
I) Trennen der zweiten manuellen medizinischen Entnahme- und Injektionsvorrichtung von dem zweiten nadellosen Konnektor (16) und hydraulisches Verbinden, mit dem ersten nadellosen Konnektor (15) des Separationsbehälters (1), einer dritten manuellen medizinischen Entnahme- und Injektionsvorrichtung,
L) Entnehmen, über die dritte manuelle medizinische Entnahme- und Injektionsvorrichtung, die mit dem ersten nadellosen Konnektor (15) verbunden ist, der dritten Flüssigkeit von dem Separationsbehälter (1) und erneutes Injizieren der dritten Flüssigkeit, so dass ein Venturi-Effekt erhalten wird, um die Thrombozyten des Sediments von Thrombozyten und die dritte Flüssigkeit erneut zu vermischen;
M) Wiederholen von Schritt L) bis zum Verschwinden des Sediments und dem Erhalt eines thrombozytenreichen Plasmas, in dem die entsprechenden Thrombozyten gleichmäßig suspendiert sind, und Aufbewahren des Plasmas in der dritten manuellen medizinischen Entnahme- und Injektionsvorrichtung.

## Revendications

1. Un récipient de séparation (1) pour la production de plasma riche en plaquettes à partir de sang, qui est stérilisable et comprenant :
- une éprouvette (11) pour une centrifugeuse de paillasse, l'éprouvette (11) ayant une portion de fond conique (12) correspondante, une première ouverture (19) qui est opposée à la portion de fond conique (12), où l'éprouvette (11) définit intérieurement un premier logement (81) correspondant ;
- un bouchon (18) qui peut être assujetti avec la première ouverture (19) de l'éprouvette (11), le bouchon (18) ayant : un premier, un deuxième et un troisième trou qui sont des trous traversants ;
- un tube (14) qui part du bouchon (18) au niveau d'un premier connecteur sans aiguille (15) de manière à ce que, lorsque le bouchon (18) est assujetti avec la première ouverture (19), le tube (14) soit disposé à l'intérieur du premier logement (81) avec une portion terminale (13) correspondante ayant une extrémité terminale correspondante, disposée distalement par rapport au bouchon (18) et au niveau de la portion de fond conique (12) de l'éprouvette (11), **caractérisé en ce que** le récipient de séparation (1) comprend également :
outre le premier connecteur sans aiguille (15) un deuxième connecteur sans aiguille (16) assujettis, respectivement, dans le premier et dans le deuxième trou pour les fermer, dans lequel, lorsque le bouchon (18) est assujetti avec la première ouverture (19), chacun des premier et deuxième connecteurs sans aiguille (15, 16) est disposé de manière à ce qu'une valve à compression correspondante soit disposée distalement par rapport à l'éprouvette (11) ;
un filtre (17) assujetti dans le troisième trou et ayant des pores de dimensions égales ou inférieures à 0,4 µm de manière à permettre uniquement le passage d'air microbiologiquement filtré conjointement avec le transfert d'un liquide à travers le premier et/ou le deuxième connecteur sans aiguille (15, 16) lorsque la valve à compression correspondante est comprimée ;
- le récipient de séparation (1) étant en outre **caractérisé en ce que** ladite portion terminale du tube (14) s'amincit vers l'extrémité correspondante de manière à obtenir un effet Venturi lorsqu'un liquide est introduit dans l'éprouvette (11), à travers le premier connecteur sans aiguille (15) et le tube (14), pour permettre le re-mélange d'un sédiment de particules et d'un liquide surnageant lorsque contenu dans le récipient de séparation (1).

2. Un kit à usage unique pour la production manuelle de plasma riche en plaquettes, comprenant :
- un récipient de séparation (1) selon la revendication 1, stérile ;
- une première poche médicale (3) pour la collecte de liquides biologiques ayant une paire correspondante de parois, opposées l'une à l'autre et définissant un deuxième logement intérieur correspondant, qui est accessible depuis une troisième ouverture (32) de la première poche médicale (3), un quatrième connecteur sans aiguille (31) disposé dans et assujetti avec la troisième ouverture (32) de manière à ce qu'une valve à compression correspondante soit disposée distalement par rapport au deuxième logement ;
- un circuit hydraulique (5) fermé et stérile, comprenant : un premier conduit hydraulique (51) ayant une première entrée (52), pouvant être raccordée hydrauliquement à un connecteur sans aiguille de manière à comprimer la valve à compression correspondante, et une première sortie, où le premier conduit hydraulique (51) ne peut être traversé par un liquide que dans une première direction (V1) de la première entrée (52) à la première sortie ; une bifurcation hydraulique disposée en aval du premier conduit hydraulique (51), par rapport à la première direction (V1), ladite bifurcation comprenant un deuxième conduit hydraulique (53) qui ne peut être traversé par un fluide que dans une seule direction (V2), concordante à la première direction (V1), vers une deuxième sortie (54) correspondante pouvant être raccordée hydrauliquement à un connecteur sans aiguille pour comprimer la valve à compression correspondante de celui-ci ; et un troisième conduit hydraulique (56) qui peut être traversé dans deux directions (V3, V4) correspondantes, depuis ou vers une troisième sortie correspondante ; où la première entrée (52) comprend un connecteur (57) correspondant pouvant être assujetti avec le quatrième connecteur sans aiguille (31) qui, lorsqu'il est assujetti, comprime la valve à compression correspondante pour raccorder hydrauliquement la première poche médicale (3) avec le premier circuit hydraulique ; et
- un premier dispositif médical (6) de collecte et d'injection manuelle, qui est stérile.

3. Un kit à usage unique pour la production manuelle de plasma riche en plaquettes selon la revendication 2, comprenant en outre :
- un deuxième dispositif médical de collecte et d'injection manuelle, qui est stérile ;
- un troisième dispositif médical de collecte et d'injection manuelle, qui est stérile et rempli d'un agent anticoagulant et
- un connecteur hydraulique femelle-femelle pour raccorder entre eux les deuxième et troisième dispositifs médicaux de collecte et d'injection manuelle, permettant l'introduction de l'agent anticoagulant dans le deuxième dispositif médical de collecte et d'injection manuelle.

4. Un kit de centrifugation réutilisable pour la production manuelle de plasma riche en plaquettes, comprenant :
- un récipient de séparation (1) selon la revendication 1 ;
- une paire de dispositifs adaptateurs (2) pour stations de centrifugation de centrifugeuses de paillasse, identiques entre eux, où chacun desdits dispositifs adaptateurs comprend : une paroi tubulaire (21) qui est extérieurement cylindrique ; chaque dispositif adaptateur (2) a un axe (23) correspondant qui est longitudinal et définit intérieurement un troisième logement (22), qui est accessible depuis une première extrémité (24) de la paroi tubulaire ; le troisième logement (22) ayant une longueur maximale (L1) correspondante qui est supérieure ou égale à une largeur maximale (L2) correspondante, mesurées le long d'un plan transversal à l'axe (23), où la paroi tubulaire (21) présente, au niveau d'un premier et d'un deuxième secteurs longitudinaux (25, 26) correspondants, opposés l'un à l'autre par rapport à l'axe (23), une première épaisseur (S1) qui est uniforme et où, au niveau d'un troisième et d'un quatrième secteurs longitudinaux (27, 28) correspondants, qui sont opposés l'un à l'autre par rapport à l'axe (23), la paroi longitudinale a une épaisseur (S2) correspondante qui n'est pas uniforme et qui est inférieure à la première épaisseur (S1), et une paroi de base qui ferme le troisième logement (22) au niveau d'une deuxième extrémité longitudinale (29) de la paroi tubulaire (21) ; chaque dispositif adaptateur (2) pouvant être inséré par la deuxième extrémité longitudinale correspondante, dans une station cylindrique d'une centrifugeuse de paillasse pour, une fois inséré, permettre de loger, dans le troisième logement (22), entre les premier et deuxième secteurs longitudinaux (25, 26), une éprouvette (11) pour centrifugeuses ayant un diamètre inférieur à la largeur maximale (L2) et ayant des dimensions telles que, lors de la centrifugation, l'éprouvette (11) reste entre les premier et deuxième secteurs longitudinaux (25, 26), ou permettre de loger, dans le troisième logement (22), une première poche médicale (3) pour la collecte de liquides, ayant des dimensions telles que, une fois au moins partiellement remplie, et une fois logée, elle s'étend du troisième au quatrième secteur longitudinal (27, 28) ;
- une deuxième autre poche médicale pour la collecte de liquides biologiques de dimensions telles que, une fois au moins partiellement remplie, et une fois logée, la deuxième poche médicale s'étend du troisième au quatrième secteur longitudinal du troisième logement (22) de l'un des dispositifs adaptateurs de la paire de dispositifs adaptateurs.

5. Un kit à usage unique pour la production manuelle d'un médicament, comprenant : un kit à usage unique pour la production manuelle de plasma riche en plaquettes selon la revendication 2 ou 3 ; un dispositif de production (85) d'un médicament comprenant du plasma riche en plaquettes, le dispositif de production (85) étant stérile et comprenant :
- une quatrième poche médicale (4) de collecte de liquides biologiques ayant une première paroi correspondante et une deuxième paroi correspondante, opposées entre elles et définissant un quatrième logement intérieur correspondant, qui est accessible depuis une deuxième ouverture (42) de la quatrième poche médicale (4),
- un troisième connecteur sans aiguille (41) disposé dans et assujetti avec la deuxième ouverture (42), de manière à ce qu'une valve à compression correspondante soit disposée distalement par rapport au quatrième logement ;
- un élément de support (43) qui comprend : une première couche de support réalisée dans un matériau biorésorbable, de préférence poreuse ; une deuxième couche, réalisée dans un deuxième matériau biorésorbable, disposée sur la première couche, où le deuxième matériau biorésorbable est une éponge polymère ou est une substance sélectionnée dans un groupe constitué de : alginate de sodium, gélatine, collagène et/ou chitosane et des combinaisons de ceux-ci et où le support médical est contenu dans le quatrième logement et est disposé avec la première couche en contact avec la première paroi et avec la deuxième couche en contact avec la deuxième paroi intérieure et, en option, un agent gélifiant.

6. Un procédé de production manuelle de plasma riche en plaquettes, le procédé étant **caractérisé en ce qu'**il comprend les étapes suivantes :
A) centrifuger le sang prélevé chez un patient, et additionné d'un agent anticoagulant, alors qu'il est contenu dans une poche médicale (3) de collecte de liquides biologiques de manière à obtenir, sur le fond de la poche médicale (3), un sédiment (33) de globules rouges et un premier liquide surnageant (34) restant ;
B) préparer un circuit hydraulique fermé et stérile, comprenant : un premier conduit hydraulique (51) ayant une première entrée (52) qui peut être hydrauliquement raccordée à un connecteur sans aiguille pour comprimer la valve à compression correspondante de celui-ci et une première sortie, où le premier conduit hydraulique (51) ne peut être traversé par un liquide que dans une première direction de l'entrée à la sortie ; une bifurcation hydraulique disposée en aval du premier conduit hydraulique, par rapport à la première direction, la bifurcation comprenant un deuxième conduit hydraulique qui ne peut être traversé par un fluide que dans une seule deuxième direction, concordante à la première direction, vers une deuxième sortie correspondante (54) pouvant être raccordée hydrauliquement à un connecteur sans aiguille, de manière à comprimer la valve à compression correspondante de celui-ci ; et un troisième conduit hydraulique qui peut être traversé dans deux directions, depuis ou vers une troisième sortie correspondante ;
C) raccorder hydrauliquement : la première entrée (52) à la poche médicale (3) ; la deuxième sortie (54) à un récipient de séparation (1) selon la revendication 1 et stérilisé ; et la troisième sortie au premier dispositif médical (6) de collecte et d'injection manuelle d'un liquide ;
D) aspirer le premier liquide surnageant (34) de la poche médicale (3), via le premier dispositif médical (6) de collecte et d'injection manuelle alors que le premier dispositif médical (6) de collecte et d'injection manuelle est raccordé à la troisième sortie pour le stocker dans le premier dispositif médical (6) de collecte et d'injection manuelle ;
E) injecter dans le circuit hydraulique fermé, via le premier dispositif médical (6) de collecte et d'injection manuelle raccordé à la troisième sortie, le premier liquide surnageant (34) qui y est stocké pour transférer le premier liquide surnageant (34) dans le récipient de séparation (1) ;
F) débrancher hydrauliquement le récipient de séparation (1) de la deuxième sortie (54) ;
G) centrifuger le premier liquide surnageant (34) alors qu'il est contenu dans le récipient de séparation (1), obtenant ainsi un sédiment de plaquettes et un deuxième liquide surnageant qui est constitué de plasma pauvre en plaquettes ;
H) raccorder hydrauliquement un deuxième dispositif médical de collecte et d'injection manuelle d'un liquide au deuxième connecteur sans aiguille (16) de manière à comprimer la valve à compression correspondante ; retourner le récipient de séparation (1) et collecter le deuxième liquide surnageant, via le deuxième dispositif médical de collecte et d'injection manuelle, et y stocker le deuxième liquide surnageant, retourner à nouveau le récipient de séparation (1) et injecter, via le deuxième dispositif médical de collecte et d'injection manuelle, une partie prédéterminée du deuxième liquide surnageant stocké, obtenant ainsi un troisième liquide contenu dans le récipient de séparation (1) ;
I) débrancher le deuxième dispositif médical de collecte et d'injection manuelle du deuxième connecteur sans aiguille (16) et raccorder hydrauliquement, au premier connecteur sans aiguille (15) du récipient de séparation (1), un troisième dispositif médical de collecte et d'injection manuelle ;
L) via le troisième dispositif médical de collecte et d'injection manuelle, raccordé au premier connecteur sans aiguille (15), collecter le troisième liquide du récipient de séparation (1) et réinjecter le troisième liquide, obtenant ainsi un effet Venturi afin de re-mélanger les plaquettes du sédiment de plaquettes et le troisième liquide ;
M) répéter l'étape L) jusqu'à la disparition du sédiment et l'obtention d'un plasma riche en plaquettes, dans lequel les plaquettes correspondantes sont uniformément suspendues, et stocker le plasma dans le troisième dispositif médical de collecte et d'injection manuelle.
